# FASCICULE DE BREVET EUROPEEN

(11) **EP 3 024 815 B1**
(45) Date de publication et mention de la délivrance du brevet: **21.08.2019**
(21) Numéro de dépôt: 14790150.8
(22) Date de dépôt: 18.07.2014
(51) Int. Cl.: A61K 8/42, A61Q 19/00, C09J 11/06, C08K 5/20, C09D 7/43, C08L 63/00

(54) **AMIDE GRAS A BASE DE LACTAME OU D'AMINOACIDE ET UTILISATION COMME ORGANOGELATEUR**
FETTAMID AUF LACTAM- ODER AMINOSÄUREBASIS UND VERWENDUNG ALS EIN ORGANISCHER GELBILDNER
LACTAME OR AMINO ACID-BASED FATTY AMIDE, AND USE AS AN ORGANOGELATOR

(30) Priorité: 25.07.2013 FR 1357349
(43) Date de publication de la demande: 01.06.2016
(73) Titulaire: Arkema France, 92700 Colombes (FR)
(72) Inventeur: BERNARD, Michael Y., F-95880 Enghien-les-bains (FR)
(74) Mandataire: Killis, Andréas
(86) Numéro de dépôt international: PCT/FR2014/051846
(87) Numéro de publication internationale: WO 2015/011376

(56) Documents cités:
- EP-A1- 2 098 502
- FR-A1- 2 281 162
- JP-A- 2011 026 573
- US-A1- 2012 129 735
- V. AJAY MALLIA ET AL: "Robust Organogels from Nitrogen-Containing Derivatives of ( R )-12-Hydroxystearic Acid as Gelators: Comparisons with Gels from Stearic Acid Derivatives +", LANGMUIR, vol. 25, no. 15, 4 août 2009 (2009-08-04), pages 8615-8625, XP055054611, ISSN: 0743-7463, DOI: 10.1021/la8042439

## Description

L'invention concerne un amide de composition spécifique comprenant dans sa composition un lactame ou un aminoacide dans un rapport spécifique par rapport à une diamine, son procédé de préparation et son utilisation comme organogélateur ou additif de rhéologie dans diverses applications, comme les compositions de revêtement, de mastic, d'agent d'étanchéité, de moulage ou de cosmétique. Ces diamides ont des performances de tenue particulière à haute température, en particulier de résistance à la coulure à haute température, de préférence à une température d'au moins 60°C et plus particulièrement à une température d'au moins 70°C.

US2012/0129735 décrit des additifs amides à base d'un monoacide carboxylique en C12-C22 et d'une amine ayant au moins deux fonctions d'amine primaire, utilisés comme additifs pour contrôler la rhéologie dans les forages pour puits de pétrole et dans une plage de températures de 4 à 49°C (40-120°F).

En effet, l'additif amide de la présente invention a une tenue thermique élevée permettant en même temps que l'activation dudit amide à des températures plus hautes, son utilisation également dans des systèmes d'application nécessitant des températures d'utilisation plus élevées. Notamment, le diamide de l'invention permet une activation et une tenue thermique et une utilisation à une température d'au moins 60°C.

La présente invention permet d'autre part par l'utilisation d'un procédé spécifique et pratique dans sa mise en oeuvre, l'incorporation fine dudit lactame ou amino-acide de manière à avoir une structure fine et bien contrôlée du diamide, en ce qui concerne ses groupements terminaux (gras), de manière à avoir une reproductibilité et un contrôle de la structure fine et par conséquent de ses performances de rhéologie.

L'invention concerne d'abord un amide de composition spécifique.

Le deuxième objet de l'invention concerne un procédé de préparation avec deux modes alternatifs selon mode A) ou selon mode B).

Un autre objet de l'invention est un organogélateur, en particulier additif de rhéologie, qui comprend ou est constitué d'au moins un amide selon l'invention.

Est également couvert, une composition de liant organique qui comprend un amide de l'invention en tant qu'organogélateur, en particulier comme additif de rhéologie, l'utilisation dudit amide dans ce but et les produits finaux ainsi obtenus.

Donc, le premier objet de la présente invention concerne un amide gras à base de (signifiant susceptible d'être obtenu par réaction de) :
a) une diamine primaire sélectionnée parmi les diamines aromatiques, cycloaliphatiques ou aliphatiques linéaires en C₂ à C₁₀,
b) un lactame ou aminoacide en C₃ à C₁₂, de préférence un lactame en C₃ à C₁₂, plus préférentiellement un lactame en C₄ à C₉ et encore plus préférentiellement en C₆,
c) une deuxième diamine primaire différente de ladite diamine a), de préférence choisie parmi les diamines aliphatiques linéaires en C₂ à C₁₀,
d) un monoacide gras hydroxylé, de préférence en C₁₈ ou C₂₀, plus préférentiellement l'acide 12-hydroxy stéarique ou l'acide 14-hydroxy eicosanoïque, de préférence l'acide 12-hydroxy stéarique,
e) en option, un monoacide non hydroxylé choisi parmi les acides aliphatiques linéaires en C₆ à C₁₂, en particulier en C₆ à C₁₀, de préférence avec le rapport molaire e/d dudit monoacide e) par rapport audit monoacide d) ne dépassant pas 0,5
et avec le rapport molaire b/(a+c) variant de 0,25 à 3/1, de préférence de 0,25 à 2/1 et plus préférentiellement de 0,35 à 2/1.

De préférence, dans la structure de l'amide de la présente invention, la formation éventuelle d'oligoamide avec des unités répétitives dérivées du lactame ou aminoacide b), par allongement de chaîne, est limitée à un nombre desdites unités répétitives ne dépassant pas 3, de préférence étant inférieur à 3 et plus particulièrement allant de 1 à 2.

Selon une option particulière de l'invention, ledit amide gras comprend au moins un diamide portant à chaque bout un groupement gras à base de monoacide d) pouvant être représenté par la formule d-b-a-d, d-b-a-b-d.

Plus particulièrement, quand ledit monoacide e) est présent, ledit amide comprend au moins trois diamides pouvant être représentés parmi les formules d-b-a-d, d-b-a-b-d, e-b-a-d, e-b-a-b-d.

Comme monoacide d) en C₁₈ ou C₂₀, on peut citer l'acide 12-hydroxy stéarique (12-HSA), l'acide 9-hydroxy stéarique (9-HSA) et/ou l'acide 10-hydroxy stéarique (10-HSA) ou l'acide 14-hydroxy eicosanoïque (14-HEA) et de préférence l'acide 12-hydroxy stéarique (12-HSA) et l'acide 14-hydroxy eicosanoïque (14-HEA), l'acide 12-hydroxy stéarique (12-HSA) étant le plus préféré. Ledit monoacide hydroxylé peut être un mélange d'au moins deux desdits monoacides d) cités.

Les diamines a) sont des amines primaires. Comme exemple de diamines aliphatiques linéaires convenables et préférées pour le composant diamine a) dudit diamide, on peut citer : l'éthylène diamine, la propylène diamine, la butylène (ou tetraméthylène) diamine, la pentaméthylène diamine, l'hexaméthylène diamine et de préférence l'éthylène diamine ou l'hexaméthylène diamine.

Comme exemple de diamines cycloaliphatiques convenables toujours selon le composant a), on peut citer: la diamine-1,3, -1,4 et -1,2 et en particulier -1,3 ou -1,4 cyclohexane, l'isophorone diamine, la bis (aminométhyl)-1,3, -1,4 ou -1,2 cyclohexane (dérivé de l'hydrogénation de respectivement m-, p-, o- xylylène diamine), de préférence la bis (aminométhyl)-1,3 ou -1,4 cyclohexane, décahydronaphtalène diamine, le bis(3-méthyl, bis (amino-4 cyclohexyl) méthane (BMACM) ou le bis (amino-4 cyclohexyl) méthane (BACM), la 1-{[4-(aminométhyl) cyclohexyl]oxy}propan-2-amine. Les diamines cycloaliphatiques préférées sont sélectionnées parmi : diamine-1,3, -1,4 cyclohexane, bis (aminométhyl)-1,3, -1,4 ou -1,2 cyclohexane, isophorone diamine et bis (amino-4 cyclohexyl) méthane.

Comme exemple convenable et préféré de diamines aromatiques en tant que composant a) dudit diamide, on peut citer : les xylylène diamines, de préférence les m- ou p- xylylène diamines, les phénylène diamines, de préférence les m- ou p-phénylène diamines ou les toluylylène diamines, de préférence les m- ou p- toluylylène diamines.

Comme exemple de diamines aliphatiques linéaires convenables et préférées pour le composant diamine c) dudit diamide, on peut citer : l'éthylène diamine, la propylène diamine, la butylène (ou tetraméthylène) diamine, la pentaméthylène diamine, l'hexaméthylène diamine et de préférence l'éthylène diamine ou l'hexaméthylène diamine.

Le composant b) peut être un lactame en C₃ à C₁₂, c'est-à-dire un cycle avec 2 à 11 atomes de carbone et un groupement amide -CO₂NH- dans le cycle où b) peut être un aminoacide équivalent, forme équivalente au lactame mais de structure linéaire au lieu de cyclique. Le composant b) est de préférence un lactame en particulier en C₄ à C₉ et plus préférentiellement en C₆ comme le caprolactame.

Comme exemple de monoacides e), on peut citer : l'acide hexanoïque, heptanoïque, octanoïque, nonanoïque, décanoïque, undécanoïque ou dodécanoïque (ou laurique). Sont préférés les acides suivants : hexanoïque, octanoïque, nonanoïque et décanoïque.

De préférence, le rapport molaire b/(a+c) entre et le lactame ou aminoacide b), en particulier lactame b), et les diamines a) et c) varie de 0,25 à 2/1 et plus préférentiellement de 0,35 à 2/1.

Selon une option particulière, ledit amide gras selon l'invention a comme diamine a) une diamine sélectionnée parmi les diamines aromatiques et plus préférentiellement dans ce cas, ladite diamine a) est une diamine aromatique parmi : xylylène diamines, de préférence m- ou p-xylylène diamines, phénylène diamines, de préférence m- ou p-phénylène diamines, toluylylène diamines, de préférence m- ou p- toluylylène diamines. Plus particulièrement, ladite diamine aromatique a) est une xylylène diamine, de préférence la m- ou p- xylylène diamine et plus préférentiellement la m-xylylène diamine. Toujours dans cette option et selon un cas plus particulier, ledit monoacide hydoxylé d), c'est-à-dire l'hydroxyacide (ou monoacide hydroxylé) en C₁₈ ou C₂₀, est l'acide 12-hydroxy stéarique (12-HSA) en l'absence dudit monoacide e). Selon une autre option alternative, ledit monoacide d) (hydroxyacide en C₁₈ ou C₂₀) est l'acide 12-hydroxy stéarique (12-HSA) en présence dudit monoacide e), de préférence e) étant sélectionné parmi l'acide hexanoïque, octanoïque, nonanoïque ou l'acide décanoïque.

Plus particulièrement, ladite diamine a) et ladite diamine c) peuvent correspondre à ou être sous forme de, un mélange de diamines composé d'une diamine aromatique a) et d'une diamine aliphatique linéaire c) ou à un mélange d'une diamine cycloaliphatique a) et d'une diamine aliphatique linéaire c).

Ledit amide selon l'invention peut être également défini comme le produit susceptible d'être obtenu par un procédé défini selon modes A) ou B) comprenant les étapes successives suivantes de réaction :
- selon mode A) :
   i) réaction de ladite diamine a) avec ledit lactame ou aminoacide b), de préférence lactame b) avec formation d'une diamine a) modifiée par b),
   ii) réaction du produit de l'étape i) avec ledit monoacide d) en présence ou absence dudit monoacide e) et en présence de ladite diamine c), ou
- selon mode B) :
   i') réaction dudit monoacide d) ou éventuellement dudit monoacide e) si présent, avec ledit lactame ou aminoacide b), de préférence lactame b), avec formation du monoacide correspondant modifié par b),
   ii') réaction dudit monoacide modifié (produit) de l'étape i') avec ladite diamine a), en présence de ladite diamine c) et si ledit monoacide de ladite étape i') est ledit monoacide d), dans ce cas, en présence ou absence dudit monoacide e), sinon, si ledit monoacide de ladite étape i') est le monoacide e), dans ce cas, en présence dudit monoacide d).

Selon une option particulière de cette définition de l'amide de l'invention selon mode B), ledit monoacide e) est présent avec les étapes successives suivantes de réaction :
i') réaction dudit monoacide e) avec ledit lactame ou aminoacide b), de préférence lactame b), avec formation d'un monoacide e) modifié par b)
ii') réaction dudit monoacide modifié formé à ladite étape i') avec ladite diamine a) et ledit monoacide d) et en présence de ladite diamine c).

Le deuxième objet de l'invention concerne un procédé correspondant de préparation de l'amide tel que défini ci-haut selon l'invention qui comprend ou est réalisé selon au moins le mode A) ou B) et lequel comprend les étapes successives suivantes de réaction comme définies ci-avant et rappelées ci-dessous.

Le procédé suivant mode A) comprend les étapes successives suivantes :
i) réaction de ladite diamine a) avec ledit lactame ou aminoacide b), de préférence lactame b) avec formation d'une diamine a) modifiée par b),
ii) réaction du produit de l'étape i) avec ledit monoacide d) en présence ou absence dudit monoacide e) et en présence de ladite diamine c).

Plus particulièrement, dans ledit procédé selon le mode A), ledit composant b) est un lactame et ladite réaction avec ladite amine a) est une oligomérisation anionique dudit lactame b) en utilisant comme amorceur anionique ladite diamine. Ce cas est particulièrement valable pour un rapport molaire b/(a+c) < 1.

Le procédé suivant mode B) comprend les étapes successives suivantes :
i') réaction dudit monoacide d) ou éventuellement dudit monoacide e) si présent, avec ledit lactame ou aminoacide b), de préférence lactame b), avec formation du monoacide correspondant modifié par b),
ii') réaction dudit monoacide modifié (produit) de l'étape i') avec ladite diamine a), en présence ou absence de ladite diamine c) et si ledit monoacide de ladite étape i') est ledit monoacide d), dans ce cas, en présence ou absence dudit monoacide e), sinon, si ledit monoacide de ladite étape i') est le monoacide e), dans ce cas, en présence dudit monoacide d).

Selon une première option de ce procédé, selon le mode B), ledit monoacide e) est présent et ledit procédé comprend les étapes successives suivantes de réaction :
i') réaction dudit monoacide e) avec ledit lactame ou aminoacide b), de préférence lactame b), avec formation d'un monoacide e) modifié par b),
ii') réaction dudit monoacide modifié formé à l'étape i') avec ladite diamine a) et ledit monoacide d) et en présence de ladite diamine c).

Un autre objet de l'invention concerne un organogélateur, lequel comprend ou est constitué d'au moins un amide tel que défini ci-haut selon l'invention ou lequel est obtenu par un procédé tel que défini ci-haut selon l'invention. Plus particulièrement, il est sous forme préactivée dans un solvant organique, de préférence dans un solvant organique comprenant au moins un solvant organique polaire.

L'invention couvre également une composition de liant organique, laquelle comprend au moins un amide ou un organogélateur tels que définis ci-haut selon l'invention.

De préférence, cette composition est une composition de revêtement, en particulier de peinture, de vernis, d'encre ou d'enduits gélifiés (« gel coat »), une composition de colle ou d'adhésif, une composition d'agent décapant, de mastic ou d'agent d'étanchéité, une composition de moulage ou une composition de cosmétique.

L'invention couvre aussi l'utilisation dudit amide en tant qu'organogélateur ou en particulier comme additif de rhéologie en milieu solvant organique et plus particulièrement dans les compositions de revêtement, de colle ou d'adhésif, d'agent décapant, de mastic ou d'agent d'étanchéité, de moulage ou de cosmétique.

Finalement, l'invention couvre un produit fini qui comprend au moins un amide comme défini selon l'invention sélectionné parmi : les revêtements, mastic ou agent d'étanchéité ou agent décapant, pièce moulée ou cosmétique, joint de colle ou d'adhésif.

Les exemples suivants sont présentés à titre d'illustration de l'invention et de ses performances, sans aucune limitation de sa couverture.

### I - Matières premières utilisées

**Tableau 1 : Matières premières utilisées**

| **Produit** | **Fonction** | **Référence commerciale** | **Fournisseur** |
|---|---|---|---|
| Hexaméthylène diamine | diamine c) | Hexaméthylène diamine 98% | Aldrich |
| Meta-xylène diamine (m-xylylène diamine) | diamine a) | mXDA | Mitsubishi Chemicals |
| Caprolactame | lactame b) | ε-Caprolactame | Aldrich |
| Acide 12-hydroxy stearique | hydroxy acide gras d) | 12-HSA | Jayant Agro |
| Carboxylate de zinc | Catalyseur | Borchikat® 22 | OMG BORCHERS SAS |
| Résine époxy | Liant | Araldite® GZ 7071X75 | Huntsman |
| Résine époxy | Liant | Araldite® GY 783 BD | Huntsman |
| Agent débullant | Agent débullant | BYK® A530 | Byk |
| Agent Dispersant | Dispersant | Disperbyk® 110 | Byk |
| Dioxyde de titane | Pigment | Tiona® 595 | Société des ocres de France |
| Oxyde de fer | Pigment | Bayferrox® 915 | Lubrizol |
| Phosphate de zinc | Pigment | ZP 10 | HEUCOPHOS |
| Talc | Additif | Finntalc® MO5 | Mondo minerals |
| Silice | charge | HPF6 | Sibelco |
| n-Butanol | Solvant | n-Butanol | Aldrich |
| Polyamide | Durcisseur | CRAYAMID® 140 | Arkema |
| Xylène | solvant | Xylène, reagent grade | Aldrich |

### II - Méthodes et tests utilisés

Les formulations sont évaluées avec deux tests : le test de résistance à l'écoulement (ou de résistance à la coulure) et une évaluation de la viscosité à différentes vitesses.

### - Test de résistance à l'écoulement

Il s'effectue à l'aide d'un contrôleur de coulure (Levelling/Sagging Tester de Sheen Instruments) qui permet d'établir la résistance d'un revêtement à la coulure due à la gravité. Fabriqué en inox et doté d'une lame droite, ce contrôleur comporte des encoches de valeur croissante.

Le test consiste à déposer différentes bandes de peinture d'épaisseur parallèle sur une bande de contraste grâce au contrôleur de coulure. La carte de contraste est immédiatement placée en position verticale, le film le plus fin en haut. L'épaisseur à laquelle les bandes se rejoignent indique la tendance à la coulure.

### - Evaluation de la viscosité

Elle s'effectue ici à l'aide d'un Brookfield® RV à 25°C (mobile : S 4). La vitesse du mobile est fixée à 50 RPM (ou rpm : rotation par minute) et l'on mesure la viscosité de chaque peinture après que celle-ci soit stabilisée. On répète l'opération pour une vitesse de 20 RPM, 10 RPM, 5 RPM, 1 RPM.

### III - Préparation et caractérisation des diamides comme organogélateurs et additifs de rhéologie)

### SYNTHESE d'un précondensat méta- (m)-xylylène diamine - caprolactame selon ledit mode A) selon l'invention

Dans un ballon de 0,5-Litre équipé d'un thermomètre, un Dean-Stark, un condensateur et d'un agitateur, on introduit sous une atmosphère d'azote 272 g de m-xylylène diamine (soit 2 moles de diamine ou 4 équivalents en amine -NH), 226 g de caprolactame (soit 2 moles) et 0,8 g de Borchikat® 22.

Le mélange est chauffé à 250°C sous courant d'azote. La réaction d'ouverture de cycle par aminolyse est contrôlée par le suivi de la viscosité. Au bout de 8 heures, la viscosité devient stable et le mélange réactionnel est refroidi à 150°C, puis déchargé.

### EXEMPLE 1 : Diamide à base d'un précondensat m-xylylène diamine - Caprolactame et d'hexaméthylène diamine

Dans un ballon de 1 litre équipé d'un thermomètre, d'un Dean-Stark, d'un condensateur et d'un agitateur, on introduit sous une atmosphère d'azote, 64,4 g de précondensat m-xylylène diamine - caprolactame (soit 0,25 moles ou 0,5 équivalents en amine, les 0,25 moles de précondensat correspondant au départ à 0,25 moles de m-XDA ayant réagi avec 0,25 moles de caprolactame), 63,8 g de hexaméthylène diamine (soit 0,25 moles, 0,5 équivalent en aminé), 315,2 g d'acide 12-hydroxy stéarique (1,00 mole, 1,00 équivalents carboxy).

Le mélange est chauffé à 200°C toujours sous courant d'azote. L'eau éliminée commence à s'accumuler dans le Dean Stark dès 150°C. La réaction est contrôlée par l'indice d'acide et d'amine. Lorsque les valeurs d'acides et d'amines sont inférieures à 10 mg KOH/g, le mélange réactionnel est refroidi à 150°C, puis déchargé dans un moule siliconé. Une fois refroidi à la température ambiante, le produit est micronisé mécaniquement par broyage et tamisage pour obtenir une granulométrie fine et contrôlée avec taille moyenne obtenue de 7 µ.

### IV - Evaluation des performances rhéologique dans une formulation de peinture

### Formulations de peintures utilisées pour l'évaluation

### 1 - Préparation

Une formulation dite « millbase » est préparée avec les proportions du tableau 3 de la manière suivante :
Dans un bol de disperseur (Dispermill® 2075 yellow line, fournisseur : Erichsen) chauffé par un système de double enveloppe :
1. Introduction des liants époxy ainsi que le dispersant et l'agent débullant. L'homogénéisation se fait après 2 minutes à 800 tours/minutes (rpm).
2. Introduction des charge et pigments puis broyage à 3000 tours/minutes pendant 30 minutes à l'aide d'une pâle de 7 cm. Grâce au bol à double enveloppe, cette étape est refroidie avec un bain d'eau froide (20°C).
3. Introduction des solvants

### 2 - Activation

24 heures après la préparation de la millbase, la formulation est de nouveau dispersée à 3000 tours/minute (rpm) à l'aide d'une pâle de 4 cm. Chaque diamide est introduit dans la millbase à une température d'activation donnée (variant de 40°C à 70°C) pendant 20 minutes à 3000 tours/minute.

Après l'ajout du durcisseur dilué (tableau 4) dans la millbase, les peintures sont ajustées avec un mélange xylène/butanol (1/1) à 0.4 Pa.s (Mesuré sur le cône 4 à 25°C à 2500 ^{s-1} à l'aide du Brookfield® CAP 1000). Les proportions entre le durcisseur et le mélange de solvants sont définies dans le tableau 4.

Après l'ajustement, la peinture est mélangée à 1500 tours/minute durant 2 minutes, puis laissée au repos pendant 30 minutes.

**Tableau 2 : formulation « Millbase »**

| **COMPOSITION de la Millbase** | **Fonction** | **% massique** |
|---|---|---|
| Araldite® GZ 7071X75 | liant | 17,3 |
| Araldite® GY 783 BD | liant | 12,9 |
| BYK® A530 | Agent débullant | 0,5 |
| Disperbyk® 110 | Dispersant | 0,5 |
| Tiona® 595 (Titanium dioxyde) | Pigment | 1,9 |
| Bayferrox® 915 595 (oxyde de fer) | Pigment | 4,1 |
| ZP 10 (phosphate de zinc) | Pigment | 7,5 |
| Finntalc® MO5 | charge | 9,4 |
| Silice HPF6 | charge | 19,0 |
| n-Butanol | Solvant | 5,4 |
| Diamide exemple 1 | Additif de Rhéologie | 0,8 |
| TOTAL | | **79,3** |

**Tableau 3 : durcisseur**

| **Composition du durcisseur** | **% en poids** |
|---|---|
| CRAYAMID® 140 | 8,8 |
| Xylène | 11,9 |
| TOTAL | **20,7** |

### 3 - Evaluation de la rhéologie des formulations et résultats

Une formulation de peinture a été réalisée suivant les proportions des tableaux 2 et 3 avec une température d'activation de 60°C suivant le protocole énoncé plus haut.

Les résultats de résistance à la coulure (tableau 4) et de rhéologie (tableau 5) montrent que le diamide de l'exemple 1 selon l'invention a un effet thixotropique sur la formulation une fois qu'elle est activée à 60°C et une bonne résistance à la coulure.

**Tableau 4 : résultats de résistance à la coulure**

| **Température d'activation** | **Diamides** | **Résistance à la coulure (µ)** |
|---|---|---|
| 60°C | 1 | 375 |

**Tableau 5 : résultats rhéologiques**

| **Température d'activation** | **Diamides** | **Viscosité Brookfield à 25°C (mPa.s)** | | | | |
|---|---|---|---|---|---|---|
| | | **1 RPM** | **5 RPM** | **10 RPM** | **50 RPM** | **100 RPM** |
| 60°C | 1 | 7800 | 2760 | 1880 | 924 | 736 |

## Revendications

1. Amide gras, **caractérisé en ce qu'**il est à base de :
a) une diamine primaire sélectionnée parmi les diamines aromatiques, cycloaliphatiques ou aliphatiques linéaires en C₂ à C₁₀,
b) un lactame ou aminoacide en C₃ à C₁₂,
c) une deuxième diamine primaire différente de ladite diamine a),
d) un monoacide gras hydroxylé,
e) en option, un monoacide non hydroxylé choisi parmi les acides aliphatiques linéaires en C₆ à C₁₂,
et **en ce que** le rapport molaire b/(a+c) varie de 0,25 à 3/1.

2. Amide gras selon la revendication 1, **caractérisé en ce que** ledit amide comprend au moins un diamide portant à chaque bout un groupement gras à base de monoacide d), ledit diamide pouvant être représenté par la formule d-b-a-d, d-b-a-b-d.

3. Amide gras selon la revendication 1 ou 2, **caractérisé en ce que** ledit monoacide e) est présent et que ledit amide comprend au moins trois parmi les diamides pouvant être représentés par les formules d-b-a-d, d-b-a-b-d, e-b-a-d, e-b-a-b-d.

4. Amide gras selon l'une des revendications 1 à 3, **caractérisé en ce que** le rapport molaire b/(a+c) est de 0,25 à 2/1.

5. Amide gras selon l'une des revendications 1 à 4, **caractérisé en ce que** ladite diamine a) est sélectionnée parmi les diamines aromatiques suivantes : xylylène diamines, phénylène diamines ou toluylylène diamines.

6. Amide gras selon la revendication 5, **caractérisé en ce que** ladite diamine est la xylylène diamine.

7. Amide selon l'une des revendications 5 ou 6, **caractérisé en ce que** ledit monoacide d) est l'acide 12-hydroxy stéarique en l'absence dudit monoacide e).

8. Amide selon l'une des revendications 5 ou 6, **caractérisé en ce que** ledit monoacide d) est l'acide 12-hydroxy stéarique en présence dudit monoacide e).

9. Amide selon l'une des revendications 1 à 8, **caractérisé en ce qu'**il est susceptible d'être obtenu par un procédé, comprenant les étapes successives suivantes selon mode A) :
i) réaction de ladite diamine a) avec ledit lactame ou aminoacide b), avec formation d'une diamine a) modifiée par b),
ii) réaction du produit de l'étape i) avec ledit monoacide d) en présence ou absence dudit monoacide e) et en présence de ladite diamine c).

10. Amide selon l'une des revendications 1 à 8, **caractérisé en ce qu'**il est susceptible d'être obtenu par un procédé, comprenant les étapes successives suivantes selon mode B) :
i') réaction dudit monoacide d) ou dudit monoacide e) si présent, avec ledit lactame ou aminoacide b) avec formation du monoacide correspondant modifié par b),
ii') réaction dudit monoacide modifié (produit) de l'étape i') avec ladite diamine a), en présence de ladite diamine c) et si ledit monoacide de ladite étape i') est ledit monoacide d), dans ce cas, en présence ou absence dudit monoacide e), sinon, si ledit monoacide de ladite étape i') est le monoacide e), dans ce cas, en présence dudit monoacide d).

11. Procédé de préparation d'un amide tel que défini selon l'une des revendications 1 à 8, **caractérisé en ce qu'**il comprend ou est réalisé selon au moins le mode A) comprenant les étapes successives suivantes de réaction :
i) réaction de ladite diamine a) avec ledit lactame ou aminoacide b), avec formation d'une diamine a) modifiée par b),
ii) réaction du produit de l'étape i) avec ledit monoacide d) en présence ou absence dudit monoacide e) et en présence de ladite diamine c).

12. Procédé selon la revendication 11 **caractérisé en ce que** dans ledit procédé selon le mode A), ledit composant b) est un lactame et que ladite réaction avec ladite amine a) est une oligomérisation anionique dudit lactame b) en utilisant comme amorceur anionique ladite diamine.

13. Procédé de préparation d'un amide tel que défini selon l'une des revendications 1 à 8, **caractérisé en ce qu'**il comprend ou est réalisé selon au moins le mode B) comprenant les étapes successives suivantes de réaction :
i') réaction dudit monoacide d) ou dudit monoacide e) si présent, avec ledit lactame ou aminoacide b), de préférence lactame b), avec formation du monoacide correspondant modifié par b),
ii') réaction dudit monoacide modifié (produit) de l'étape i') avec ladite diamine a), en présence de ladite diamine c) et si ledit monoacide de ladite étape i') est ledit monoacide d), dans ce cas, en présence ou absence dudit monoacide e), sinon, si ledit monoacide de ladite étape i') est le monoacide e), dans ce cas, en présence dudit monoacide d).

14. Procédé selon la revendication 13, **caractérisé en ce que** ledit monoacide e) est présent avec ledit procédé comprenant les étapes successives suivantes de réaction :
i') réaction dudit monoacide e) avec ledit lactame ou aminoacide b), avec formation d'un monoacide e) modifié par b),
ii') réaction dudit monoacide modifié formé à l'étape i') avec ladite diamine a) et ledit monoacide d) et en présence de ladite diamine c).

15. Organogélateur, **caractérisé en ce qu'**il comprend ou est constitué d'au moins un amide tel que défini selon l'une des revendications 1 à 10 ou obtenu par un procédé tel que défini selon l'une des revendications 11 à 14.

16. Organogélateur selon la revendication 15, **caractérisé en ce qu'**il est sous forme préactivée dans un solvant organique.

17. Composition de liant organique, **caractérisée en ce qu'**elle comprend au moins un amide tel que défini selon l'une des revendications 1 à 10 ou un organogélateur tel que défini selon la revendication 15 ou 16.

18. Composition selon la revendication 17, **caractérisée en ce qu'**il s'agit d'une composition de revêtement, d'une composition de colle ou d'adhésif, une composition d'agent décapant, de mastics ou d'agents d'étanchéité, une composition de moulage ou une composition de cosmétique.

19. Utilisation d'un amide tel que défini selon l'une des revendications 1 à 10 ou obtenu par un procédé tel que défini selon la revendication 11 à 14, en tant qu'organogélateur.

20. Utilisation selon la revendication 19, **caractérisée en ce qu'**elle concerne les compositions de revêtement, de colle ou d'adhésif, de mastic, d'agent décapant ou d'agent d'étanchéité, de moulage ou de cosmétique.

21. Produit fini, **caractérisé en ce qu'**il comprend au moins un amide tel que défini selon l'une des revendications 1 à 10 ou obtenu par un procédé tel que défini selon l'une des revendications 11 à 14, ledit produit étant sélectionné parmi : revêtement, mastic, agent d'étanchéité, agent décapant, pièce moulée, cosmétique, joint de colle ou d'adhésif.

## Patentansprüche

1. Fettamid, **dadurch gekennzeichnet, dass** es auf Folgendem basiert:
a) einem primären Diamin, ausgewählt aus aromatischen, cycloaliphatischen oder geraden aliphatischen C₂- bis C₁₀-Diaminen,
b) einem C₃- bis C₁₂-Lactam oder einer C₃- bis C₁₂-Aminosäure,
c) einem zweiten primären Diamin, das von dem Diamin a) verschieden ist,
d) einer hydroxylierten Monofettsäure,
e) gegebenenfalls einer nicht-hydroxylierten Monosäure, ausgewählt aus geraden aliphatischen C₆-bis C₁₂-Säuren,
und dadurch, dass das Molverhältnis b/(a+c) von 0,25 bis 3/1 variiert.

2. Fettamid nach Anspruch 1, **dadurch gekennzeichnet, dass** das Amid mindestens ein Diamid umfasst, das an jedem Ende eine Fettgruppe auf der Basis der Monosäure d) trägt, wobei das Diamid durch die Formel d-b-a-d, d-b-a-b-d dargestellt werden kann.

3. Fettamid nach Anspruch 1 oder 2, **dadurch gekennzeichnet, dass** die Monosäure e) vorhanden ist und dass das Amid mindestens drei der Diamide umfasst, die durch die Formeln d-b-a-d, d-b-a-b-d, e-b-a-d, e-b-a-b-d dargestellt werden können.

4. Fettamid nach einem der Ansprüche 1 bis 3, **dadurch gekennzeichnet, dass** das Molverhältnis b/(a+c) 0,25 bis 2/1 beträgt.

5. Fettamid nach einem der Ansprüche 1 bis 4, **dadurch gekennzeichnet, dass** das Diamin a) aus den folgenden aromatischen Diaminen ausgewählt ist: Xylylendiaminen, Phenylendiaminen oder Toluylylendiaminen.

6. Fettamid nach Anspruch 5, **dadurch gekennzeichnet, dass** das Diamin Xylylendiamin ist.

7. Amid nach einem der Ansprüche 5 oder 6, **dadurch gekennzeichnet, dass** die Monosäure d) in Abwesenheit der Monosäure e) 12-Hydroxystearinsäure ist.

8. Amid nach einem der Ansprüche 5 oder 6, **dadurch gekennzeichnet, dass** die Monosäure d) in Gegenwart der Monosäure e) 12-Hydroxystearinsäure ist.

9. Amid nach einem der Ansprüche 1 bis 8, **dadurch gekennzeichnet, dass** es durch ein Verfahren erhältlich ist, das gemäß dem Modus A) die nachstehenden aufeinanderfolgenden Schritte umfasst:
i) Umsetzen des Diamins a) mit dem Lactam oder der Aminosäure b) unter Bildung eines durch b) modifizierten Diamins a),
ii) Umsetzen des Produkts von Schritt i) mit der Monosäure d) in Gegenwart oder Abwesenheit der Monosäure e) und in Gegenwart des Diamins c).

10. Amid nach einem der Ansprüche 1 bis 8, **dadurch gekennzeichnet, dass** es durch ein Verfahren erhältlich ist, das gemäß dem Modus B) die nachstehenden aufeinanderfolgenden Schritte umfasst:
i') Umsetzen der Monosäure d) oder der Monosäure e), wenn vorhanden, mit dem Lactam oder der Aminosäure b) unter Bildung der entsprechenden durch b) modifizierten Monosäure,
ii') Umsetzen der modifizierten Monosäure (des Produkts) von Schritt i') mit dem Diamin a) in Gegenwart des Diamins c) und, wenn die Monosäure von Schritt i') die Monosäure d) ist, dann in Gegenwart oder Abwesenheit der Monosäure e), anderenfalls, wenn die Monosäure von Schritt i') die Monosäure e) ist, dann in Gegenwart der Monosäure d).

11. Verfahren zur Herstellung eines Amids nach einem der Ansprüche 1 bis 8, **dadurch gekennzeichnet, dass** es mindestens den Modus A) umfasst oder mindestens gemäß diesem durchgeführt wird, der die nachstehenden aufeinanderfolgenden Reaktionsschritte umfasst:
i) Umsetzen des Diamins a) mit dem Lactam oder der Aminosäure b) unter Bildung eines durch b) modifizierten Diamins a),
ii) Umsetzen des Produkts von Schritt i) mit der Monosäure d) in Gegenwart oder Abwesenheit der Monosäure e) und in Gegenwart des Diamins c).

12. Verfahren nach Anspruch 11, **dadurch gekennzeichnet, dass** in dem Verfahren gemäß dem Modus A) die Komponente b) ein Lactam ist und dass die Umsetzung mit dem Amin a) eine anionische Oligomerisation des Lactams b) unter Verwendung des Diamins als anionischer Starter ist.

13. Verfahren zur Herstellung eines Amids nach einem der Ansprüche 1 bis 8, **dadurch gekennzeichnet, dass** es mindestens den Modus B) umfasst oder mindestens gemäß diesem durchgeführt wird, der die nachstehenden aufeinanderfolgenden Reaktionsschritte umfasst:
i') Umsetzen der Monosäure d) oder der Monosäure e), wenn vorhanden, mit dem Lactam oder der Aminosäure b), vorzugsweise mit dem Lactam b), unter Bildung der entsprechenden durch b) modifizierten Monosäure,
ii') Umsetzen der modifizierten Monosäure (des Produkts) von Schritt i') mit dem Diamin a) in Gegenwart des Diamins c) und, wenn die Monosäure von Schritt i') die Monosäure d) ist, dann in Gegenwart oder Abwesenheit der Monosäure e), anderenfalls, wenn die Monosäure von Schritt i') die Monosäure e) ist, dann in Gegenwart der Monosäure d).

14. Verfahren nach Anspruch 13, **dadurch gekennzeichnet, dass** die Monosäure e) in dem Verfahren vorhanden ist, das die nachstehenden aufeinanderfolgenden Reaktionsschritte umfasst:
i') Umsetzen der Monosäure e) mit dem Lactam oder der Aminosäure b) unter Bildung einer durch b) modifizierten Monosäure e),
ii') Umsetzen der in Schritt i') gebildeten modifizierten Monosäure mit dem Diamin a) und der Monosäure d) und in Gegenwart des Diamins c).

15. Organogelbildner, **dadurch gekennzeichnet, dass** er mindestens ein Amid nach einem der Ansprüche 1 bis 10 umfasst oder daraus besteht oder durch ein Verfahren nach einem der Ansprüche 11 bis 14 erhalten worden ist.

16. Organogelbildner nach Anspruch 15, **dadurch gekennzeichnet, dass** er in präaktivierter Form in einem organischen Lösungsmittel vorliegt.

17. Organische Bindemittelzusammensetzung, **dadurch gekennzeichnet, dass** sie mindestens ein Amid nach einem der Ansprüche 1 bis 10 oder einen Organogelbildner nach Anspruch 15 oder 16 umfasst.

18. Zusammensetzung nach Anspruch 17, **dadurch gekennzeichnet, dass** es sich um eine Beschichtungszusammensetzung, eine Leim- oder Klebstoffzusammensetzung, eine Beizmittel-, Kitt- oder Dichtungsmittelzusammensetzung, eine Gusszusammensetzung oder eine Kosmetikzusammensetzung handelt.

19. Verwendung eines Amids nach einem der Ansprüche 1 bis 10 oder erhalten durch ein Verfahren nach einem der Ansprüche 11 bis 14 als Organogelbildner.

20. Verwendung nach Anspruch 19, **dadurch gekennzeichnet, dass** sie Beschichtungs-, Leim- oder Klebstoff-, Kitt-, Beizmittel- oder Dichtungsmittel-, Guss- oder Kosmetikzusammensetzungen betrifft.

21. Endprodukt, **dadurch gekennzeichnet, dass** es mindestens ein Amid nach einem der Ansprüche 1 bis 10 umfasst oder durch ein Verfahren nach einem der Ansprüche 11 bis 14 erhalten worden ist, wobei das Produkt ausgewählt ist aus: Beschichtung, Kitt, Dichtungsmittel, Beizmittel, Formteil, Kosmetik, Leim- oder Klebstoffverbindung.

## Claims

1. Fatty amide, **characterized in that** it is based on:
a) a primary diamine selected from aromatic, cycloaliphatic or linear C₂ to C₁₀ aliphatic diamines,
b) a C₃ to C₁₂ lactam or amino acid,
c) a second primary diamine different from the said diamine a),
d) a hydroxylated fatty monoacid,
e) optionally, a nonhydroxylated monoacid chosen from linear C₆ to C₁₂ aliphatic acids,
and **in that** the molar ratio b/(a+c) varies from 0.25 to 3/1.

2. Fatty amide according to Claim 1, **characterized in that** the said amide comprises at least one diamide carrying, at each end, a fatty group based on monoacid d), it being possible for the said diamide to be represented by the formula d-b-a-d or d-b-a-b-d.

3. Fatty amide according to Claim 1 or 2, **characterized in that** the said monoacid e) is present and **in that** the said amide comprises at least three among the diamides which may be represented by the formulae d-b-a-d, d-b-a-b-d, e-b-a-d and e-b-a-b-d.

4. Fatty amide according to one of Claims 1 to 3, **characterized in that** the molar ratio b/ (a+c) is from 0.25 to 2/1.

5. Fatty amide according to one of Claims 1 to 4, **characterized in that** the said diamine a) is selected from the following aromatic diamines: xylylenediamines, phenylenediamines or toluylenediamines.

6. Fatty amide according to Claim 5, **characterized in that** the said diamine is xylylenediamine.

7. Amide according to either of Claims 5 and 6, **characterized in that** the said monoacid d) is 12-hydroxystearic acid in the absence of the said monoacid e).

8. Amide according to either of Claims 5 and 6, **characterized in that** the said monoacid d) is 12-hydroxystearic acid in the presence of the said monoacid e).

9. Amide according to one of Claims 1 to 8, **characterized in that** it is capable of being obtained by a process comprising the following successive stages according to mode A):
i) reaction of the said diamine a) with the said lactam or amino acid b), with formation of a diamine a) modified by b),
ii) reaction of the product from stage i) with the said monoacid d) in the presence or absence of the said monoacid e) and in the presence of the said diamine c).

10. Amide according to one of Claims 1 to 8, **characterized in that** it is capable of being obtained by a process comprising the following successive stages according to mode B):
i') reaction of the said monoacid d) or of the said monoacid e), if present, with the said lactam or amino acid b), with formation of the corresponding monoacid modified by b),
ii') reaction of the said modified monoacid (product) from stage i') with the said diamine a) in the presence of the said diamine c) and, if the said monoacid of the said stage i') is the said monoacid d), in this case in the presence or absence of the said monoacid e); if not, if the said monoacid of the said stage i') is the monoacid e), in this case in the presence of the said monoacid d).

11. Process for the preparation of an amide as defined according to one of Claims 1 to 8, **characterized in that** it comprises or is carried out according to at least the mode A) comprising the following successive reaction stages:
i) reaction of the said diamine a) with the said lactam or amino acid b), with formation of a diamine a) modified by b),
ii) reaction of the product from stage i) with the said monoacid d) in the presence or absence of the said monoacid e) and in the presence of the said diamine c).

12. Process according to Claim 11, **characterized in that**, in the said process according to the mode A), the said component b) is a lactam and **in that** the said reaction with the said amine a) is an anionic oligomerization of the said lactam b) using the said diamine as anionic initiator.

13. Process for the preparation of an amide as defined according to one of Claims 1 to 8, **characterized in that** it comprises or is carried out according to at least the mode B) comprising the following successive reaction stages:
i') reaction of the said monoacid d) or of the said monoacid e), if present, with the said lactam or amino acid b), preferably lactam b), with formation of the corresponding monoacid modified by b),
ii') reaction of the said modified monoacid (product) from stage i') with the said diamine a) in the presence of the said diamine c) and, if the said monoacid of the said stage i') is the said monoacid d), in this case in the presence or absence of the said monoacid e); if not, if the said monoacid of the said stage i') is the monoacid e), in this case in the presence of the said monoacid d).

14. Process according to Claim 13, **characterized in that** the said monoacid e) is present with the said process comprising the following successive reaction stages:
i') reaction of the said monoacid e) with the said lactam or amino acid b), with formation of a monoacid e) modified by b),
ii') reaction of the said modified monoacid formed in stage i') with the said diamine a) and the said monoacid d) and in the presence of the said diamine c).

15. Organogelator, **characterized in that** it comprises or consists of at least one amide as defined according to one of Claims 1 to 10 or obtained by a process as defined according to one of Claims 11 to 14.

16. Organogelator according to Claim 15, **characterized in that** it is in preactivated form in an organic solvent.

17. Organic binder composition, **characterized in that** it comprises at least one amide as defined according to one of Claims 1 to 10 or an organogelator as defined according to Claim 15 or 16.

18. Composition according to Claim 17, **characterized in that** it is a coating composition, an adhesive composition, a stripping agent, mastic or sealant composition, a moulding composition or a cosmetic composition.

19. Use of an amide as defined according to one of Claims 1 to 10 or obtained by a process as defined according to one of Claims 11 to 14, as organogelator.

20. Use according to Claim 19, **characterized in that** it relates to coating, adhesive, mastic, stripping agent or sealant, moulding or cosmetic compositions.

21. Finished product, **characterized in that** it comprises at least one amide as defined according to one of Claims 1 to 10 or obtained by a process as defined according to one of Claims 11 to 14, the said product being selected from: coating, mastic, sealant, stripping agent, moulded part, cosmetic or adhesive seal.
